# EUROPEAN PATENT SPECIFICATION

(11) **EP 3 481 396 B1**
(45) Date of publication and mention of the grant of the patent: **31.05.2023**
(21) Application number: 17740488.6
(22) Date of filing: 30.06.2017
(51) Int. Cl.: A61K 31/452, A61K 45/06, A61P 29/00

(54) **THERAPY OF BACTERIAL INFECTIONS.**
THERAPIE BAKTERIELLER INFEKTIONEN
TRAITEMENT DE L'INFECTION BACTÉRIENNES

(30) Priority: 08.07.2016 GB 201611900; 17.02.2017 GB 201702601
(43) Date of publication of application: 15.05.2019
(73) Proprietor: Selectimmune Pharma AB, 222 42 Lund (SE)
(72) Inventor: SVANBORG, Catharina, 211 33 Malmo (SE); BUTLER, Daniel Sebastian Christopher, 223 62 Lund (SE); AMBITE, Ines, 223 62 Lund (SE)
(74) Representative: Greaves Brewster LLP
(86) International application number: PCT/IB2017/053976
(87) International publication number: WO 2018/007920

(56) References cited:
- WO-A1-2009/000038
- CAMPBELL D J; TENIS N; ROSAMILIA A; CLEMENTS J A; DWYER P L: "Urinary levels of substance P and its metabolites are not increased in interstitial cystitis.", BJU INTERNATIONAL, vol. 87, 2001, pages 35-38, XP002773414,
- MARTINEZ ALEJANDRA N ET AL: "Antagonist of the neurokinin-1 receptor curbs neuroinflammation in ex vivo and in vitro models of Lyme neuroborreliosis", JOURNAL OF NEUROINFLAMMATION, BIOMED CENTRAL LTD., LONDON, GB , vol. 12, no. 1 30 December 2015 (2015-12-30), page 243, XP009520091, ISSN: 1742-2094, DOI: 10.1186/S12974-015-0453-Y Retrieved from the Internet: URL:https://link.springer.com/article/10.1 186/s12974-015-0453-y

## Description

The present invention relates to compositions for treating bacterial infections. in particular acute cystitis, and to compositions for use in these therapies.

### Background of the Invention

Urinary tract infections (UTIs) are common and may be dangerous. The clinical presentation and severity varies, depending on the site of infection and molecular basis of disease. In acute pyelonephritis (APN), bacteria ascend into the renal pelvis, where they cause an intense mucosal inflammatory response with progression into the renal parenchyma. Symptoms include high fever, malaise, loin pain as well as poor feeding and irritability in infants. In acute cystitis, infection is localized to the urinary bladder, resulting in dysuria, frequency and supra-pubic pain, typically without systemic involvement. While the clinical entities of acute pyelonephritis and acute cystitis typically are quite distinct, the molecular determinants of clinical presentation and severity are largely unknown.

In acute pyelonephritis, a pathogen-specific TLR4 response is activated by P fimbriated *E. coli,* through ceramide release and the successive phosphorylation of the TICAM-1 (TRIF) and TICAM-2 (TRAM) adaptors, CREB-1, c-FOS and c-JUN activates IRF- and AP1- dependent transcription. Additional involvement of MyD88, TIRAP and NF-kB depends on the virulence repertoire of the infecting strain. Genetic studies in the murine UTI model have identified IRF3-dependent gene expression and mCXCR2-dependent neutrophil activation as determinants of bacterial clearance and tissue homeostasis. Infected *Irf3*^{*-*/*-*} or *mCxcr2*^{*-*/*-*} mice develop severe APN and tissue damage after one week and relevance for human APN susceptibility has been demonstrated, through disease-associated *IRF3* and *CXCR1* polymorphisms in APN prone patients.

The cause of acute cystitis susceptibility has not been defined, however and it is rapidly becoming a therapeutic enigma as antibiotic resistance is reducing the options for treatment to a minimum. There is a great, unmet need for alterative therapies. Genetic markers of APN susceptibility show no association to acute cystitis, emphasizing the differences in pathogenesis and genetic control. Acute uncomplicated cystitis is characterized by localized inflammation and symptoms from the lower urinary tract, typically dysuria and urgency and frequency of urination. It occurs predominantly in girls and healthy women with normal urinary tracts and at least 60% of all females will report an episode during their lifetime. The recurrence rate is high, especially in a subset of patients, where recurrent cystitis episodes may cause chronic tissue damage and impact the quality of life. In addition, acute cystitis patents pose a highly significant challenge to the health care system.

The urinary bladder mucosa is often under microbial attack but does not always retaliate with such force, as in acute cystitis. In asymptomatic carriers, the epithelium remains fairly unresponsive, despite bacterial numbers well above 10⁵ CFU/ml (9, 10). It is therefore challenging to understand, at the molecular level, how a state of exaggerated inflammation can be generated specifically in acute cystitis patients. Interactions of bladder cells with pathogenic bacteria have been shown to trigger an innate immune response, involving the epithelium and adjacent mucosal cells, such as mast cells and macrophages. Uroepithelial cells from women with recurrent cystitis have an increased density of receptors for adhering bacteria and bacterial persistence in intracellular communities has been studied as a reservoir of infection, but the molecular basis of susceptibility and disease has remained unclear.

Infections are accompanied by characteristic symptoms from the site of infection and by general malaise, in case of systemic involvement. Pain serves as a key indicator of disease severity and as a warning signal for the host. Pain is also one of the classical hallmarks of inflammation, together with hyperemia, edema and increased temperature in inflamed tissue foci. Infections are accompanied by pain, as the nervous system is engaged, either indirectly or directly by bacterial attack on nerve cells. Bladder pain characterizes acute cystitis, suggesting that the nervous system is critically involved.

Neuropeptides and receptors implicated in nociception and pain signalling include Substance P (SP), its receptor NK1R as well as Galanin and its receptors, which affect nociceptive signalling in the dorsal horn and the dorsal root ganglia. Recently, bacteria were shown to activate sensory neurons directly, through interactions with specific virulence factors. Neurons express receptors for bacterial toxins, such as globotriaosylceramide that binds Shiga toxins and P fimbriae and for TLR4 that recognizes lipopolycsaccharide in the context of co-receptors like MD2 (LPS) . Neuropeptides and their receptors have also been shown to regulate inflammation and to modulate immune responses, after release into infected tissues.

In addition to pain (dysuria), characteristic clinical symptoms include urgency and frequency of urination, caused by involuntary contractions of the bladder detrusor muscles. The molecular basis of these symptoms is not known but the symptom profile suggests that bacteria activate a mucosal neuropeptide response involving SP and NK1R, which in addition to pain, also control smooth muscle contraction. Interestingly, it has been speculated that the bladder epithelium shares key features with sensory neurons, including the ability to express neuropeptides and neuropeptide receptors suggesting that symptoms of acute cystitis are caused by the combined activation of the epithelial barrier and mucosal nerve cells. Studies of pelvic pain have identified the O antigen of LPS as a microbial agonist and NK1R signaling as a host determinant of pain induced by pseudorabies virus.

The applicants have investigated whether acute cystitis is accompanied by a mucosal neuropeptide- and neuropeptide receptor response. This investigation followed on from the discovery that neuropeptides and neuropeptide receptors were among the significantly regulated gene families in samples from patients with bacteriuria.

Some neuropeptide inhibitors such as P2X3 and NK1 receptor antagonists have been implicated previously in animal models for interstitial cystitis. However, this is a quite different disease, being chronic and not of bacterial origin, and therefore findings in these models would not be expected to be transferable to acute cystitis.

### Summary of the Invention

The applicants have shown that acute cystitis isolates trigger a strong neuropeptide- and neuropeptide receptor response in human neuronal cells and bladder epithelial cells and confirm these observations in the murine UTI model. Furthermore, pharmacologic inhibition of the NK1R receptor has been found to rescue highly susceptible mice from acute cystitis, suggesting that the neuropeptide response also drives bladder inflammation. Consistent with recent findings described in copending International Patent Application No. PCT PCT/IB2016/050070, an extreme, neuropeptide hyper-activation state in *Asc*^{*-*/*-*} and *Nlrp3*^{*-*/*-*} mice that develop severe, IL-1β driven acute cystitis was found, and this effect could be inhibited by treating susceptible mice with an IL-1R inhibitor Anakinra. However, this work provides a new molecular basis for the symptoms of acute cystitis, and identifies NK1R inhibition as a potential therapeutic complement to antibiotics, which may have a broad spectrum anti-infection application. Furthermore, in view of the nature of the inhibition, the treatment may be used in the management of cystitis-related pain.

According to the present invention there is provided an antagonist of Neurokinin-1 receptor (NK1R), or its ligand Substance P (SP), for use in the treatment of a bacterial infection or the management of pain caused by a bacterial infection.

In a particular embodiment, the infection is cystitis is acute cystitis. In particular, the treatment is for the treatment of acute cystitis or for the management of acute cystitis-related pain.

In another particular embodiment the antagonist is an antagonist of NK1R, such as SR140333 (1-[2-[(3*S*)-3-(3,4-Dichlorophenyl)-1-[2-[3-(1-methylethoxy)phenyl]acetyl]-3-piperidinyl]ethyl]-4-phenyl-1-azoniabicyclo[2.2.2]octane chloride), Aprepitant (5-([(2*R*,3*S*)-2-((*R*)-1-[3,5-bis(trifluoromethyl)phenyl]ethoxy)-3-(4-fluorophenyl)morpholino]methyl)-1*H*-1,2,4-triazol-3(2*H*)-one), Fosaprepitant ([3-{[(2*R*,3*S*)-2-[(1*R*)-1-[3,5-bis(trifluoromethyl)phenyl] ethoxy]-3-(4-fluorophenyl)morpholin-4-yl]methyl}-5-oxo- 2*H*-1,2,4-triazol-1-yl]phosphonic acid), Casopitant((2*S*,4*S*)-4-(4-Acetyl-1-piperazinyl)-*N*-[(1*R*)-1-[3,5-bis(trifluoromethyl)phenyl]ethyl]-2-(4-fluoro-2-methylphenyl)-*N*-methyl-1-piperidinecarboxamide), L-733,060 ((2*S*,3*S*)-3-{[3,5-bis(trifluoromethyl)benzyl]oxy}-2-phenylpiperidine), Vestipitant (2*S*)-*N*-[(1*R*)-1-[3,5-bis(trifluoromethyl)phenyl]ethyl]-2-(4-fluoro-2-methylphenyl)-*N*-methylpiperazine-1-carboxamide), Maropitant((7*R*,8*S*)-*N*-[(5-*tert*-Butyl-2-methoxyphenyl)methyl]-7-[di(phenyl)methyl]-1-azabicyclo[2.2.2]octan-8-amine), as well as CP99994 ((2*S*,3*S*)-*N*-[(2-Methoxyphenyl)methyl]-2-phenyl-3-piperidinamine) and L703.606 ((2R,3R)-2-benzhydryl-N-[(2-iodanylphenyl)methyl]-1-azabicyclo[2.2.2]octan-3-amine).

A particular example of an antagonist of NK1R is SR140333.

Other particular examples are CP99994 and L703.606.

For administration to patients, the antagonist is suitably administered in the form of a pharmaceutical composition, which further comprise a pharmaceutically acceptable carrier. Such compositions are known in the art.

Suitable pharmaceutical compositions will be in either solid or liquid form. They may be adapted for administration by any convenient route, such as parenteral, oral or topical administration or for administration by inhalation or insufflation. The pharmaceutical acceptable carrier may include diluents or excipients which are physiologically tolerable and compatible with the active ingredient.

Parenteral compositions are prepared for injection, for example either subcutaneously or intravenously. They may be liquid solutions or suspensions, or they may be in the form of a solid that is suitable for solution in, or suspension in, liquid prior to injection. Suitable diluents and excipients are, for example, water, saline, dextrose, glycerol, or the like, and combinations thereof. In addition, if desired the compositions may contain minor amounts of auxiliary substances such as wetting or emulsifying agents, stabilizing or pH-buffering agents, and the like.

Oral formulations will be in the form of solids or liquids, and may be solutions, syrups, suspensions, tablets, pills, capsules, sustained-release formulations, or powders. Oral formulations include such normally employed excipients as, for example, pharmaceutical grades of mannitol, lactose, starch, magnesium stearate, sodium saccharin, cellulose, magnesium carbonate, and the like.

Topical formulations will generally take the form of suppositories or intranasal aerosols. For suppositories, traditional binders and excipients may include, for example, polyalkylene glycols or triglycerides; such suppositories may be formed from mixtures containing the active ingredient.

The amount of reagent administered will vary depending upon factors such as the nature of the reagent being used, the size and health of the patient, the nature of the condition being treated etc. in accordance with normal clinical practice. Typically, a dosage in the range of from 1 µg-50mg/Kg for instance from 2-20 mg/Kg, such as from 5-15 mg/Kg would be expected to produce a suitable effect.

The antagonist of the invention may be administered alone or in combination with another therapeutic agent, such as an IL-1β inhibitors and MMP inhibitors, or proteins selected from ASC or NLRP-3. Such reagents are described in co-pending patent application no PCT/IB2016/050070. The components of any combination may be administered together or sequentially, in accordance with required clinical practice.

In a further aspect, the invention provides a composition for treating a bacterial infection, such as acute cystitis, or for managing pain caused by a bacterial infection, said method comprising administering to a patient in need thereof, an effective amount of a reagent selected from the group consisting of an antagonist of Neurokinin-1 receptor (NK1R), or its ligand Substance P (SP).

In a further aspect, there is provided a method for diagnosing cystitis and in particular, acute cystitis, said method comprising detecting elevated levels of SP in urine of a subject.

The urinary bladder is lined by a specialized transitional epithelial cell layer, which maintains mucosal integrity under conditions of varied urine volumes and bladder wall extension. The submucosal smooth muscle cells define bladder tonus and are engaged by the nervous system to effectuate bladder voiding. Bladder innervation has been extensively studied, not least in patients suffering from neurogenic bladder disorders, caused by mechanic lesions or neurological disease. Here we propose that the pain response and involuntary muscle contractions in acute cystitis are caused by a direct bacterial effect on nerve cells and epithelial cells in the urinary bladder mucosa, resulting in the activation of neuropeptides and neuropeptide receptors known to regulate pain and involuntary muscle contractions. We propose that this response is a key to the clinical symptoms of acute cystitis and suggest that NK1R and SP might be targeted therapeutically, to alleviate the symptoms of acute cystitis.

In addition to regulating circuits in the mucosal nervous system, the neuropeptides and their receptors regulated the inflammatory response to infection. This was demonstrated by pharmacologic inhibition of the NK1R receptor *in vivo,* in mice susceptible to acute cystitis. The NK1R inhibitor was shown to attenuate the IL-1β-driven, hyperinflammatory response in *Nlrp3*^{*-*/*-*} mice and to reduce the pathology score, suggesting that the NK1R/SP loop controls important aspects of innate immunity and mucosal inflammation. In addition, SP and NK1R levels were lowered in uninfected mice, suggesting that NK1R is important to maintain a homeostatic neuropeptide environment in mucosal tissues. The results agree with previous studies, showing that pro-inflammatory cytokines, including IL-1β, are induced by SP/NK1R signaling. Interestingly, IL-1β has also been shown to induce NK1R expression, suggesting that neurokrine and innate immune responses converge onto this pathway. In both cases, activation has been proposed to involve the transcription factor NF-□B, suggesting that NF-□B may serve as a potent upstream regulator of the neurokrine and innate immune responses that define the pathology and symptomology of acute cystitis.

The applicants successfully mapped key genes that control the neuropeptide response to infection in this model, using mice with single gene deletions. Interestingly, the genes that controlled the NK1R and SP responses were recently identified as genes that control the susceptibility to acute cystitis, through an IL-1b-driven response network. *Nlrp3*^{*-*/*-*} and *Asc*^{*-*/*-*} mice suffer from an IL-1β hyper-activation disorder, which leads to excessive bladder inflammation. In the absence of a functional inflammasome, IL-1β is processed by MMP-7, a metalloproteinase that cleaves pro-IL-1β in the mucosa. ASC and NLRP3 were shown to act as repressors of the *Mmp7* promoter, explaining the excessive MMP-7 activation in *Nlrp3*^{*-*/*-*} and *Asc*^{*-*/*-*} mice. Like MMP-7, NK1R and SP tissue levels increased in *Nlrp3*^{*-*/*-*} and *Asc*^{*-*/*-*} mice, in epithelial cells and the mucosal nerve plexus. We did not detect an increase in the transcription of NK1R and SP in the mice, however, suggesting that the increased tissue levels do not reflect a direct effect on *Nk1r (Tac1r)* or *Ppt1* gene expression. The low levels of NK1R and SP in *IL1b*^{*-*/*-*} mice and the marked reduction after therapeutic inhibition with the IL-1R antagonist Anakinra^{R}, suggest that IL-1α and β serve as essential regulators of NK1R and SP responses to infection. In addition, the levels of NK1R and SP were reduced in uninfected *IL1b*^{*-*/*-*} mice, suggesting that IL-1β may control the mucosal neuropeptide homeostasis. The absence of NK1R and SP in mucosal tissues of *Tlr4*^{*-*/*-*} mice further emphasized that sensing by TLR4 is important for both the neurocrine and the innate immune responses to infection.

Infection activated mucosal GALR2 expression, as shown by an increase in tissue staining and by QRTPCR, suggesting a role for GAL2R in the sensing of pathogen attack. Mucosal GALR2 responses to mucosal infection have not been studied but GALR2 has been shown to control the response to nociceptive stimuli in the dorsal horn. Depending on the stimulus, GALR2 may either activate or inactivate pain-signaling. Yamamoto et al. Endocrine 2011; 40(3); 400-407**,** proposed that the GALRs may be involved in the regulation of innate immunity as the two receptors, GALR1 and GALR3, support inflammation in human keratinocytes, by inducing IL-1β and IL-8. In the acute cystitis model, infection caused a dramatic increase in GAL2R expression, with involvement of both epithelial cells and nerve cells. We show that this response is unrelated to the pro-inflammatory pathway controlled by *TLR4*/*Il1b* and not directly related to the mechanism of bladder pathology, defined by *Il1b, Asc* and *Nlrp3.* The results suggest that GALR2 recognises infection and interacts with the innate immune response through a separate mechanism.

A link between the neuropeptide response, symptoms of acute cystitis and pelvic pain has been suggested, based on the increase in urine SP concentrations in postmenopausal women with overactive bladders and pelvic pain. Furthermore, it has been shown that the bladder pain syndrome is accompanied by an increase in SP producing nerve fibers in the urinary bladder in patients and rats. SP has also been proposed to support the development of bladder pathology, in patients with interstitial cystitis or bladder pain syndrome. In these patients, an increase was observed in SP-reactive varicose nerve fibers within the lamina propria. Consistent with these studies, we detected elevated SP and NK1R levels in patients with acute cystitis compared to patients with ABU. Furthermore, in a longitudinal study of ABU, a pair-wise, intra-individual comparison of SP levels revealed a difference between asymptomatic and symptomatic episodes in individual patients. Based on the present study, the applicants have identified that neuropeptides, in particular NK1R and SP as biomarkers and therapeutic targets in patients with acute cystitis or other infection-related bladder pain syndromes. Short-term NK1R inhibition therapy might be desirable, in order to avoid possible side-effects.

The invention will now be described by way of example with reference to the accompanying Figures.

### Description of the Figures

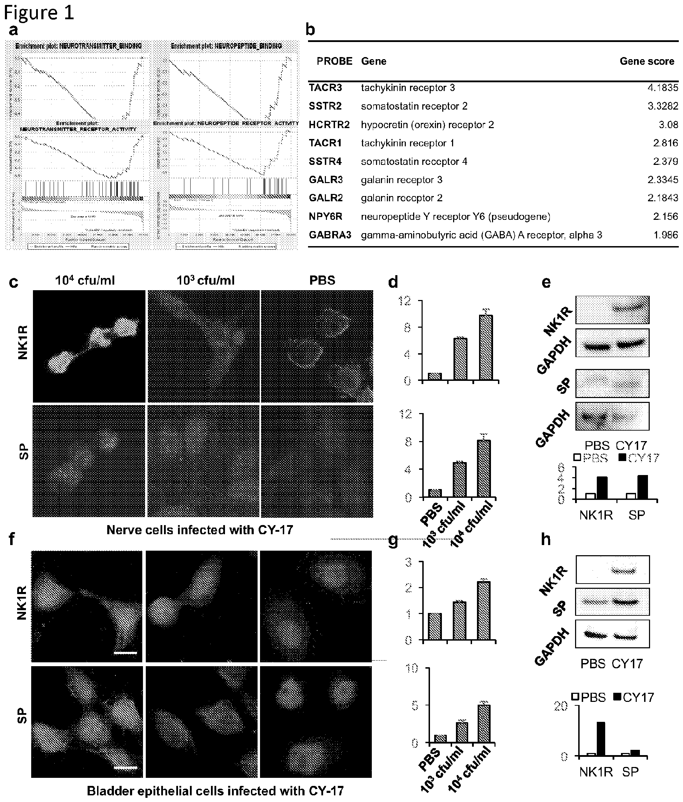

**Figure 1****. Bacteria activates a neuropeptide response**
   Genes involved in neural processes were regulated in patients with ABU, as defined by whole genome transcriptomic analysis. (a) Neurotransmitter and neuropeptide-related gene sets were enriched, 48 hours after inoculation of PI with the ABU strain E. coli 83972. Regulated genes were involved in neurotransmitter binding, neurotransmitter receptor activity, neuropeptide binding and neuropeptide receptor activity. Gene Set Enrichment Analysis (GSEA) of significantly regulated genes, defined in comparison with the pre-inoculation data set in the same patient. (b) The most strongly regulated genes involved in neural processes, defined by GSEA in four patients, using samples from five time points post inoculation (*n* = 20, 3, 24, 48 hours, 1 and 2 weeks). (c) NK1R and SP responses to in vitro infection of human nerve cells (SH-SY5Y) with the acute cystitis strain CY-17 are dose-and time-dependent. NK1R and SP staining is shown. (d) Fluorescence intensities for (c), means + SEMs of 50 cells, one of three experiments, (e) Western blot confirming the dose dependent NK1R and SP responses in infected nerve cells, quantified in the histogram, compared to PBS controls. One of three experiments (f) NK1R and SP responses to in vitro infection of human bladder epithelial cells (HTB9) with the acute cystitis strain CY-17 are dose-and time-dependent. NK1R and SP staining is shown. One of three experiments (g) Fluorescence intensities in (e), quantified as means + SEMs of 50 cells, one of three experiments (h) Western blot confirming the dose dependent increase in NK1R and SP in infected bladder epithelial cells, quantified in the inserted histogram, compared to PBS controls. *** = p < 0.001, two-tailed t-test.

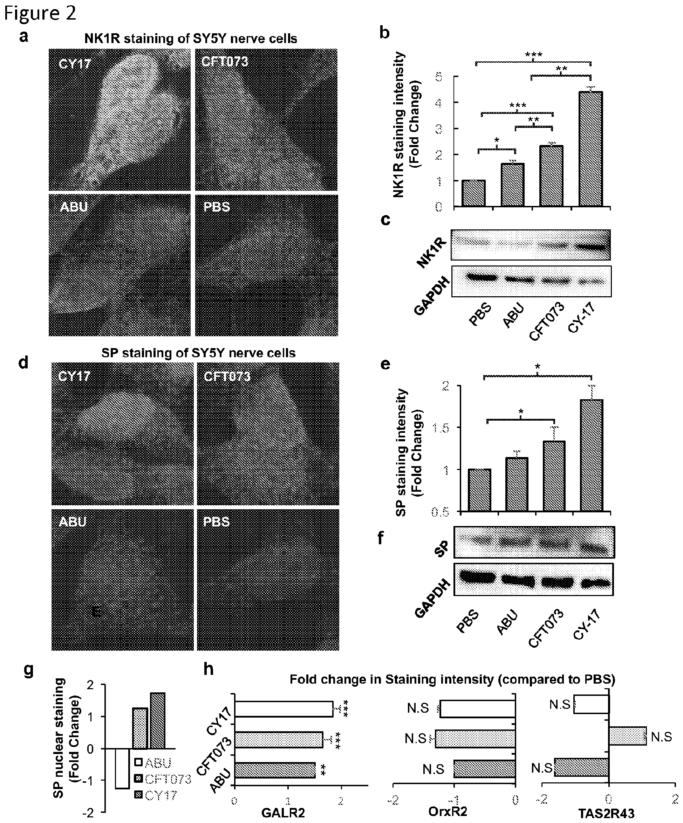

**Figure 2****. Bacterial virulence influences the neuropeptide response in nerve cells**
   SH-SY5Y nerve cells were infected with the acute cystitis strain CY-17, the pyelonephritis strain CFT073 or the asymptomatic bacteriuria strain *E.coli 83972* and stained for NK1R or SP. **(a)** Difference in staining intensity, identifying CY-17 as the most potent activator of NK1R (one of three experiments). **(b)** Quantification of staining intensity, compared to the uninfected control, *n* = 50 cells, one of two experiments **(c)** Western blot of NK1R protein levels in nerve cells after *in vitro* infection with CY-17, CFT073 or ABU one of two experiments). **(d)** Difference in staining intensity, identifying CY-17 as the most potent activator of SP (one of three experiments). **(e)** Quantification of staining intensity in **(d),** *n* = 50 cells, one of three experiments, **(f)** Western blot of SP protein levels in nerve cells after *in vitro* infection with CY-17, CFT073 or ABU (one of three experiment). **(g)** Quantification of SP nuclear staining in nerve cells infected with CY-17, CFT073 or ABU, *n* = 50 cells, one of two experiments. **(h)** Quantification of neuropeptide receptors GALR2 (left), OrxR2 (middle) and TAS2R43 (right) in nerve cells infected with CY-17, CFT073 or ABU compared to uninfeced controls, n = 50 cells, of of three experiments. Means ± SEMs, N.S = non-significant, * *P* < 0.05, ** < 0.01 and *** *P* < 0.001, two-tailed unpaired *t*-test.

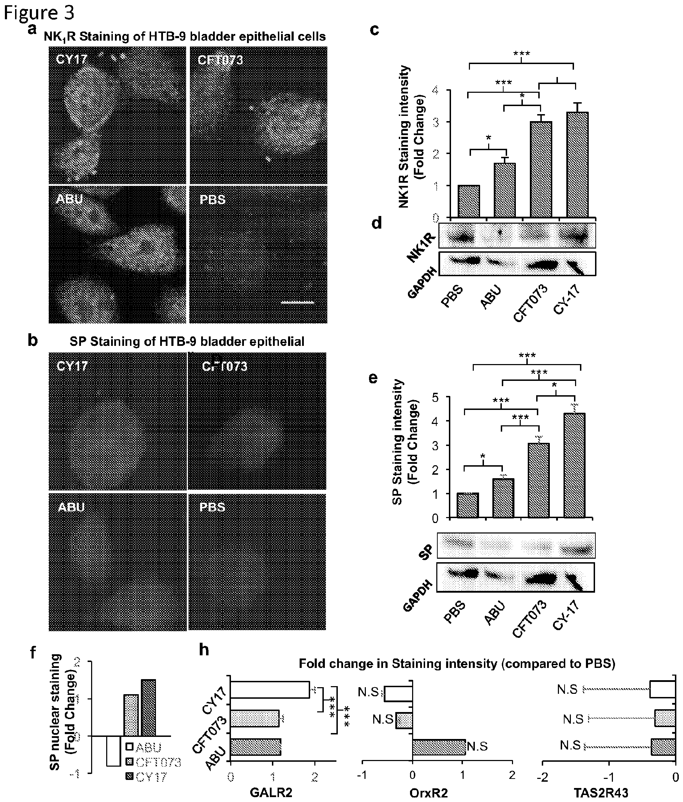

**Figure 3****. Bacterial virulence influences the neuropeptide response in bladder epithelial cells**
   HTB9 bladder epithelial cells were infected with the acute cystitis strain CY-17, the pyelonephritis strain CFT073 or the asymptomatic bacteriuria strain *E.coli 83972* and stained for NK1R or SP. **(a)** Difference in staining intensity, identifying CY-17 as the most potent activator of NK1R, one of three experiments, **(b)** Quantification of staining intensity, compared to the uninfected control, *n* = 50 cells, one of three experiments. **(c)** Western blot of NK1R protein levels in nerve cells after *in vitro* infection with CY-17, CFT073 or ABU, one of two experiments **(d)** Difference in staining intensity, identifying CY-17 as the most potent activator of SP one of three experiments, **(e)** Quantification of staining intensity in compared to the uninfected controls, *n* = 50 cells, one of three experiments. **(f)** Western blot of SP protein levels in nerve cells after *in vitro* infection with CY-17, CFT073 or ABU, one of two experiments **(g)** Quantification of SP nuclear staining in nerve cells infected with CY-17, CFT073 or ABU, *n* = 50 cells, one of three experiments, **(h)** Quantification of neuropeptide receptors GALR2 (left), OrxR2 (middle) and TAS2R43 (right) in nerve cells infected with CY-17, CFT073 or ABU compared to uninfected controls control, *n* = 50 cells, one of three experiments. Means ± SEMs, N.S = non-significant, * *P* < 0.05, ** < 0.01 and *** *P* < 0.001, two-tailed unpaired *t*-test.

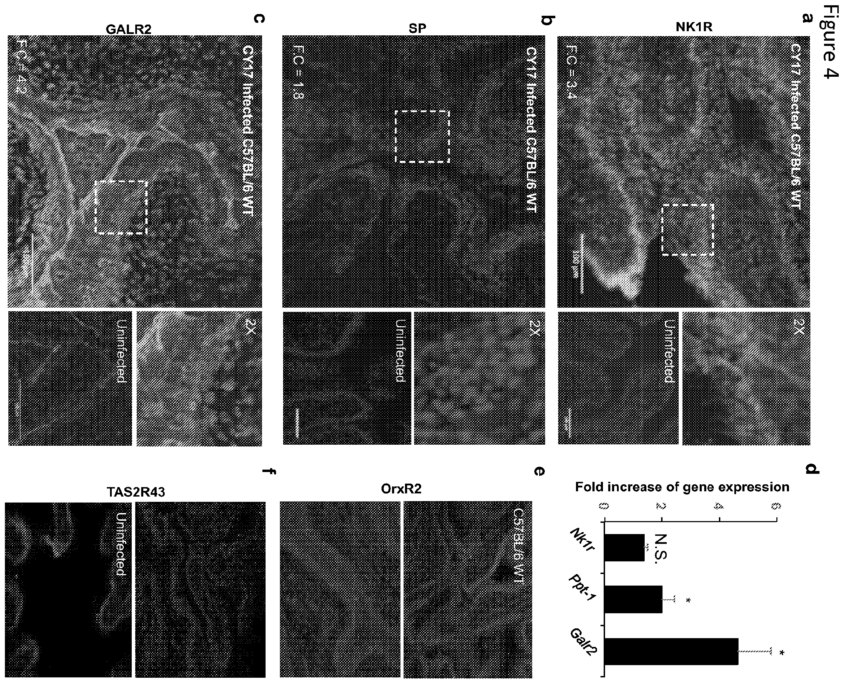

**Figure 4****. Neuropeptide response to bladder infection in the murine cystitis model**
   Mucosal NK1R, SP and GALR2 staining in bladder sections from CY-17 infected C57BL/6 WT mice, 7 days after infection. Responses to infection are quantified as the fold change, compared to uninfected WT mice, which are used as controls. **(a)** Mucosal NK1R staining, fold change in lower left corner, one of three experiments, **(b)** Mucosal SP staining, fold change in lower left corner one of three experiments, **(c)** Mucosal GALR2 staining, fold change in lower left corner, **(d)** Quantification of *Nk1R, Ppt-1* and *Galr2* gene expression, by qRT-PCR normalized against the uninfected controls for each gene (*n* = 2 mice per group), one of two experiments **(e)** Mucosal OrxR2 staining in bladder sections compared to uninfected controls one of two experiments. **(f)** TAS2R43 staining in bladder sections compared to uninfected controls one of two experiments. Means ± SEMs, * = p < 0.05, two tailed *t*-test, *n* = 4 mice per group.

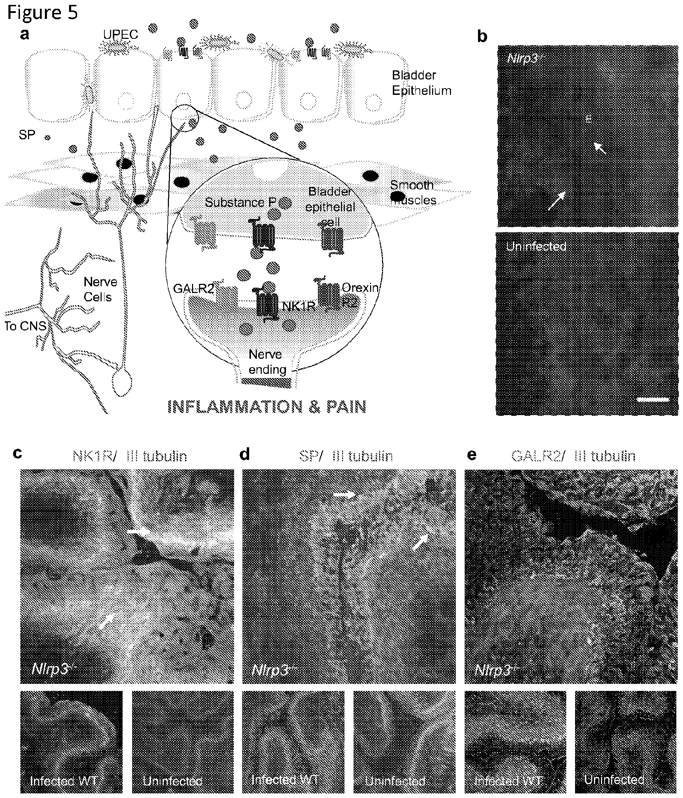

**Figure 5****. Cellular origin of the neuropeptide and neuropeptide receptor response**
   Identification of nerve cells (βIII tubulin) in bladder tissue sections from CY-17 infected or uninfected mice. Tissues were obtained 7 days after infection and susceptible *Nlrp3*^{*-*/*-*}mice were compared to infected C57BL/6 WT mice. The sections were co-stained for NK1R, SP and GALR2. **(a)** Model of the mucosal response to infection, focusing on neuropeptides and their receptors. Images are based on results from the present study. Acute cystitis strains activate an NK1R response in the epithelial lining of the bladder and in nerve cells, located between the epithelial cells baso-laterally, and in the mucosal nerve plexus, with axons extending the spinal cord and central nervous system. The mutual response to these agonists creates an amplifying neuronal circuit, leading to pain and involuntary contractions of the lamina muscularis. **(b)** βIII Staining of nerve cells in bladder tissue sections from *Nlrp3*^{*-*/*-*} mice, and uninfected *Nlrp3*^{*-*/*-*} mice, one of three experiments. **(c)** Co-localization of βIII tubulin with NK1R in bladder tissue sections from *Nlrp3*^{*-*/*-*} mice, infected WT mice and uninfected *Nlrp3*^{*-*/*-*} mice, white arrows indicating co-localization between NK1R and nerve cells, of two experiments. **(d)** Co-localization of βIII tubulin with SP in bladder tissue sections from *Nlrp3*^{*-*/*-*} mice, infected WT mice and uninfected *Nlrp3*^{*-*/*-*} mice, white arrows indicating co-localization between SP and nerve cells one of two experiments, **(e)** Co-localization of βIII tubulin with GALR2 in tissue sections from infected *Nlrp3*^{*-*/*-*} mice, infected WT mice and uninfected *Nlrp3*^{*-*/*-*} mice. Data from two experiments, two genotypes, infected and uninfected mice, 7 days. N= 3 mice per group, a total of 24 mice.

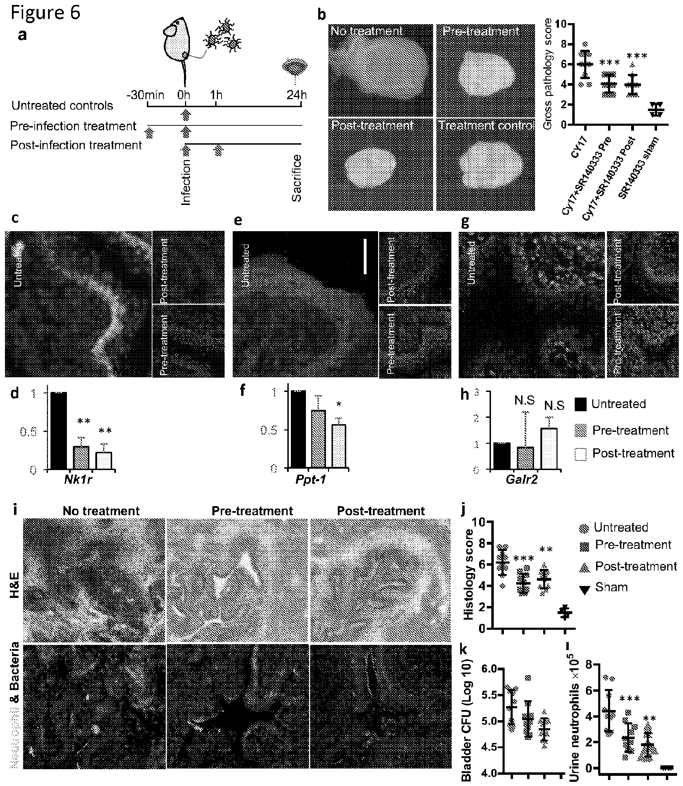

**Figure 6****. Therapeutic effect of inhibiting NK1R in *Nlrp3*^{*-*/*-*} mice**
   To inhibit the NK1R dependent response to infection, we used the NK1R antagonist SR 140333. **(a)** Experimental scheme of NK1R antagonist (SR140333) treatment. Susceptible *Nlrp3*^{*-*/*-*} mice were infected with CY-17 for 24 hours, and the neuropeptide response and disease severity was compared between untreated infected mice, treated and infected mice and sham treated uninfected mice. The mice were either pre-treated with SR140333 i.p. for 30 minutes prior to infection or treated i.p. 1 hour after infection. Sham treated mice received SR140333 i.p. and PBS intravesically. *n* = 10 mice in each treatment group, n=6 in the sham treated, uninfected group. N=2 in the uninfected control group. Means + SEMs of 2 experiments, total number of mice = 39. **(b)** Protection against gross bladder pathology in SR140333 treated mice compared untreated, infected mice, **(c)** Inhibition of NK1R staining in treated mice compared to infected mice not receiving SR140333. Sections were obtained from 4 mice per condition. **(d)** Inhibition of *Nk1r* expression, detected by qRT-PCR analysis of treated mice compared to untreated, infected mice. Sections were obtained from 5 mice per treatment group. **(e)** Attenuated SP staining in SR140333 treated infected mice compared to untreated mice (*n* = 4 mice per condition). **(f)** Inhibition of *Ppt-1* gene expression detected in mice receiving SR140333 post infection but not in the pretreatment group, compared to untreated, infected mice. *n* = 5 mice per treatment group **(g)** SR140333 does not effect GALR2 staining in infected mice. *n* = 4 mice per condition. **(h)** *Galr2* gene expression is not altered after treatment with SR140333. *n* = 5 mice per treatment group (* = p < 0,05, ** = p < 0.01, Mann-Whitney test). **(i)** Protection from bladder tissue pathology shown in H&E stained and neutrophil and bacterial immunostained sections from treated versus control mice. **(j)** Histology score showing reduced pathology after treatment with SR140333 (k) Kinetics of bacterial clearance in the urine of SR140333 treated mice (**l**) Kinetics of neutrophil recruitment in the urine of SR140333 treated mice, compared to untreated mice. *(n* = 10 mice per treatment group, *n* = 4 mice per sham group, means ± SEMs, * *P* < 0.05, ** *P* < 0.01, *** *P* < 0.001, one way Anova).

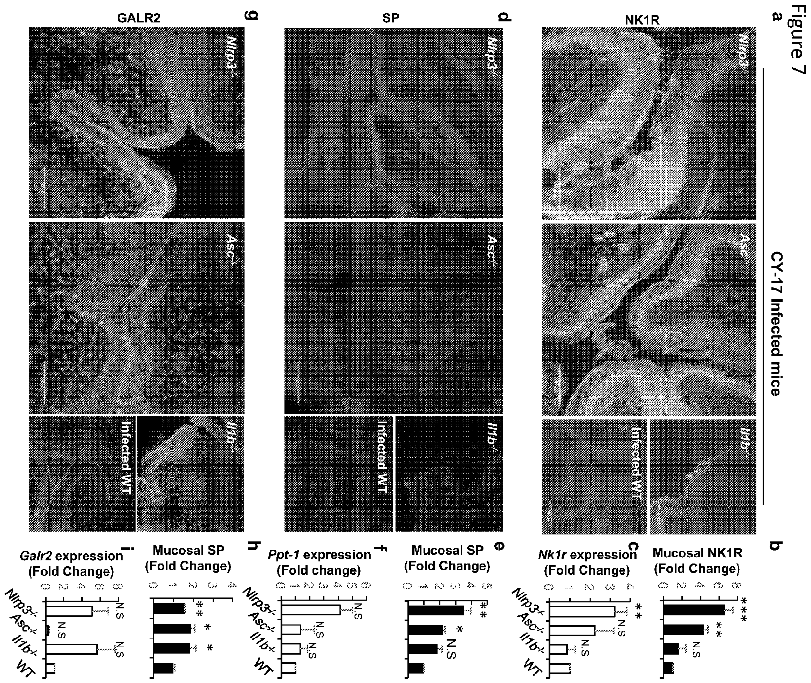

**Figure 7****. Genetic control of mucosal neuropeptide response to CY-17 infection**
   Genes controlling the NK1, SP or GALR2 response to infection were identified using mice with single gene deletions affecting the susceptibility to *E*. *coli* infection and acute cystitis. The mice were infected with CY-17 and tissues were obtained after 7 days. **(a)** Strong mucosal NK1R staining in bladder sections from *Nlrp3*^{*-*/*-*} and *Asc*^{*-*/*-*} mice, compared to WT mice. No increase in mucosal NK1R staining in *Il1b*^{*-*/*-*} mice, compared to WT mice. **(b)** Quantification of mucosal NK1R response to infection in *Nlrp3*^{*-*/}*⁻, Asc*^{*-*/*-*} WT and *Il1b*^{*-*/*-*}mice. **(c)** *Nk1r* expression in *Nlrp3*^{*-*/}*⁻, Asc*^{*-*/}*⁻, Il1b*^{*-*/*-*} and WT mice. **(d)** Strong mucosal SP staining in *Nlrp3*^{*-*/*-*} and *Asc*^{*-*/*-*} bladder sections compared to WT mice. No increase in mucosal SP staining in *Il1b*^{*-*/*-*} mice, compared to WT mice. **(e)** Quantification of mucosal SP response to infection in*Nlrp3*^{*-*/}*⁻, Asc*^{*-*/}*⁻, Il1b*^{*-*/*-*} and WT mice. **(f)** *Ppt-1* expression in *Nlrp3*^{*-*/}*⁻, Asc*^{*-*/}*⁻, Il1b*^{*-*/*-*} and WT mice. **(g)** Increase in mucosal and submucosal GALR2 staining in *Nlrp3*^{*-*/*-*} and *Asc*^{*-*/}*⁻, Il1b*^{*-*/*-*} bladder sections, **(h)** Quantification of the GALR2 response to infection in *Nlrp3*^{*-*/}*⁻, Asc*^{*-*/}*⁻, Il1b*^{*-*/*-*} and WT mice. **(i)** *Galr2* gene expression in *Nlrp3*^{*-*/}*⁻, Asc*^{*-*/}*⁻, Il1b*^{*-*/*-*} and WT mice. Sections were obtained from 4 mice per group. qRT-PCR included total bladder RNA from 2 mice per group, one of two experiments is shown. * = p < 0.05, ** = p < 0.01, *** p < 0.001, two tailed *t*-test).

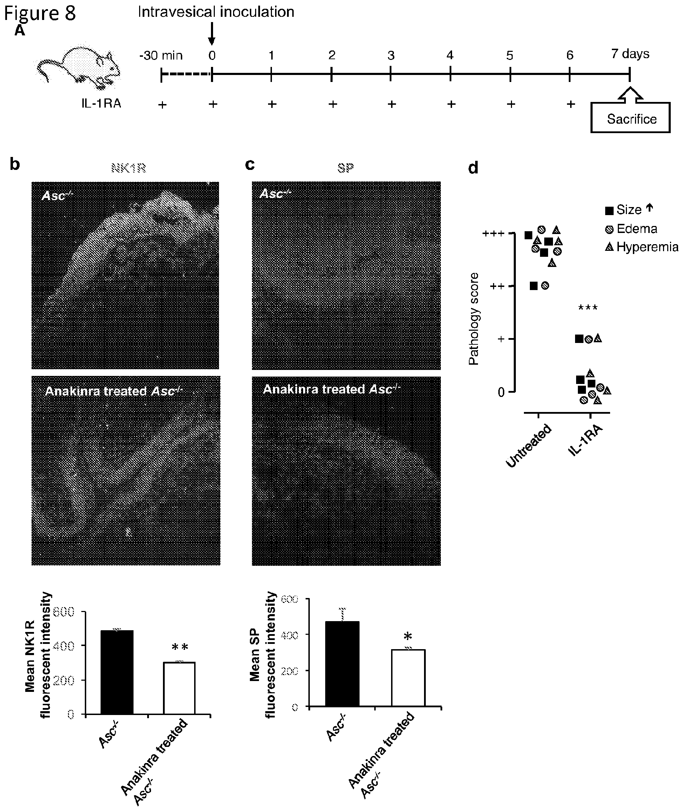

**Figure 8****. Anakinra^{R} treatment attenuates the NK1R and SP response in infected mice. (a)** The mice were pre-treated with interleukin 1 receptor antagonist (IL1-RA) Anakinra, 30 min before infection and daily after infection with *E. coli* CFT073 (1 mg in 100 µl of PBS i.p. per mouse). **(b)** Reduction in NK1R in Anakinra^{R} treated compared to untreated control mice. Bladder tissue sections from infected *Asc*^{*-*/*-*} mice treated with Anakinra^{R} were stained for NK1R. Histogram shows mean fluorescent intensity of mucosal NK1R expression. **(c)** Reduction in SP staining in Anakinra^{R} treated *Asc*^{*-*/*-*} mice compared to untreated mice. Histogram shows mean fluorescent intensity of mucosal SP expression. Sections were obtained from 3 mice per group; one of two experiments is shown. * = p < 0.05, ** = p < 0.01, two tailed *t*-test). **(d)** The disease severity score was reduced by the Anakinra (*n* = 3-4, *** *P* < 0.001, compared to untreated *Asc*^{*-*/*-*} mice, Fisher's exact test, Reference for (d):.

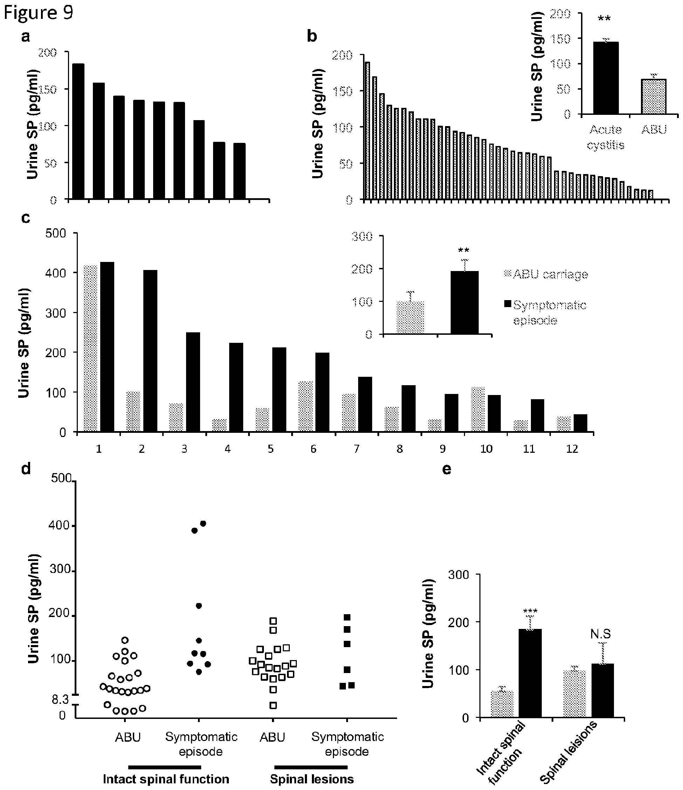

**Figure 9****. Urine SP levels in patients with acute cystitis or asymptomatic bacteriuria**
   Urine samples were obtained from patients diagnosed with acute cystitis or patients with long-term ABU and analysed for SP concentrations, by ELISA. **(a)** Urine SP levels in patients with acute cystitis, n = 9. **(b)** Urine SP levels in patients with long-term ABU carriage (*n* = 40). Histogram insert shows the median SP concentrations in acute cystitis and ABU, medians + SEMs (** = p < 0.01, Wilcoxon signed rank test). **(c)** Elevated SP levels in urine during symptomatic episodes compared to asymptomatic episodes in the same patient. Paired urine samples (*n* = 12) from patients during asymptomatic carriage compared to SP levels during symptomatic episodes. The histogram shows a pair-wise comparison between the two carrier states (** = p < 0.01, Wilcoxon signed rank test). **(d)** Effect of spinal cord injury on urine SP levels. Difference between patients with an intact cord and patients with spinal lesions (*** = p < 0.001, Mann-Whitney test).

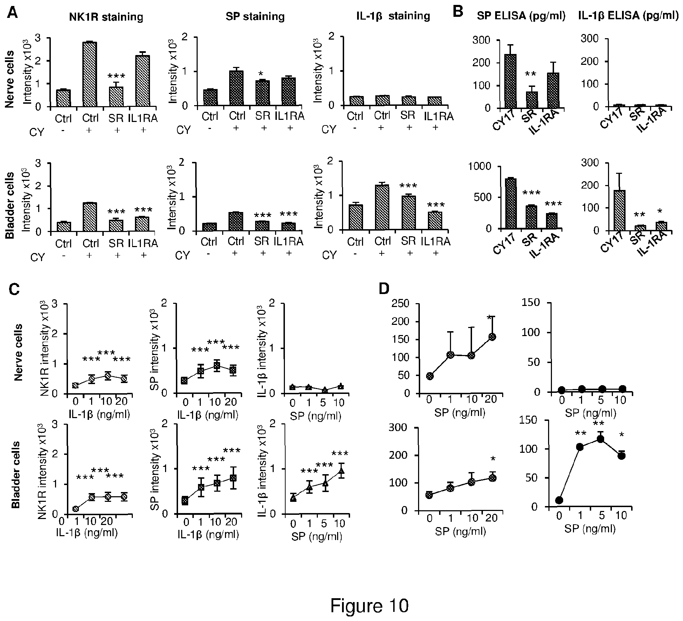

**Figure 10****: In vitro effects of NK1R antagonists and IL-1RA**
   HTB-9 cells and differentiated SH-SY5Y cells, pre-treated with SR140333, or IL-1RA cells were infected with CY17 and the NK1R, SP and IL-1β response was quantified by **(A)** confocal imaging. **(B)** IL-1β- and SP secretion was quantified by ELISA, in culture supernatants. These results were confirmed by ELISA showing a decrease in SP secretion in both nerve- and epithelial cells. IL-1β secretion was inhibited in epithelial cells by both SR140333 and IL-1RA **(****Figure 10 C-D**) (^{∗} = *P* < 0.05, ** *= P* < 0.01, *** = *P* < 0.001, Two-tailed t-test for confocal imaging data and Mann-Whitney U-test for ELISAs.

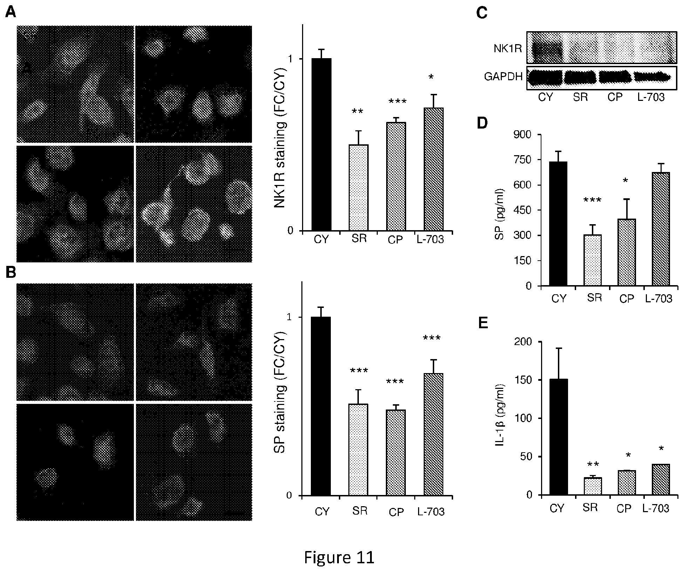

**Figure 11****: NK1R antagonists decrease NK1R, SP and IL-1b i*n vitro***
   HTB9 bladder epithelial cells were pretreated with three different NK1R antagonists (SR140333, CP99994 and L703.606) to evaluate the effect on NK1R, SP and IL-1b **(A)** NK1R Staining and quantification of staining intensity in NK1R antagonist treated cells **(B)**) SP Staining and quantification of staining intensity in NK1R antagonist treated cells. **(C)** Western blot confirmation of (A). **(D)** Levels of secreted SP was decreased by NK1R antagonist treatment **(E)** IL-1b secretion was also decreased by NK1R antagonist treatment Scale bars = 20 µm.

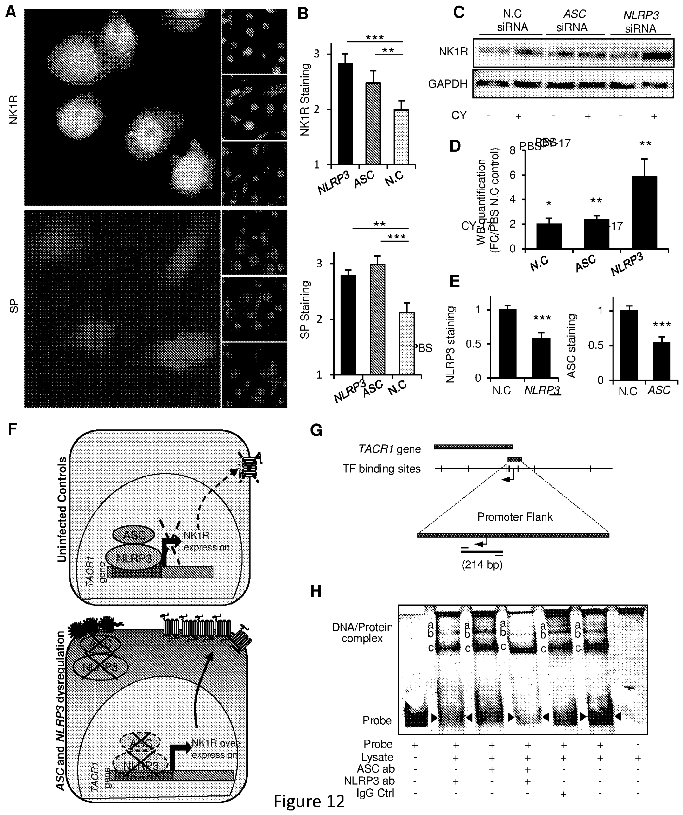

**Figure 12****: *NLRP3 and ASC acts as repressors of NK1R and SP***
   To confirm the *in vivo* NK1R and SP increase in *Asc*^{*-*/*-*} and *Nlrp3*^{*-*/*-*} mice, we created an *in vitro* system using *NLRP3* and *ASC* SiRNA in bladder epithelial cells and investigated the effects on NK1R and SP after infection (10⁴ cfu/ml, four hours). **(A)** Confocal imaging showing a marked increase of NK1R and SP staining in *ASC* and *NLRP3* siRNA transfected cells compared to scrambled negative control siRNA (N.C). **(B)** Quantification of staining intensity from (A) defined as fold change to scrambled negative control PBS (Mean + SEM of 50 cells, ** = *P* < 0.01, *** = *P* < 0.001, two tailed t-test). **(C)** Western blot analysis of whole cell lysates confirming the increase seen in (A) **(D)** Quantification of band intensities from the Western blot in (C) (Mean + SEM of three blots, * *= P* < 0.05, ** *= P* < 0.01 two tailed t-test. **(E)** The gene-silencing efficiency of siRNA was determined by quantifying ASC and NLRP3 by confocal imaging in *ASC* and *NLRP3* siRNA treated cells compared to negative control siRNA treated cells. **(F)** Model showing how ASC/NLRP3 complex binds to the *TACR1* promoter, leading to a moderate NK1R expression during infection. However, when *ASC* or *NLRP3* is not present, *TACR1* is de-repressed, leading to increased NK1R expression. **(G)** PCR amplification of a 214 bp fragment in the *TACR1* promoter. **(H)** Electric mobility shift assay (EMSA) of the 214 bp *TACR1* promoter fragment showing three band shifts after treatment with uninfected cell extracts. Competition with antibodies against NLRP3 showed a pronounced decrease in the intensity of the upper band (Band a) and a decrease in the intensity of the second shifted band (Band b). Competition with ASC antibody did not show any clear results, but combination of the two antibodies showed a complete loss of the upper band (band a) and even a further decrease of band b (1 of 3 representative EMSAs).

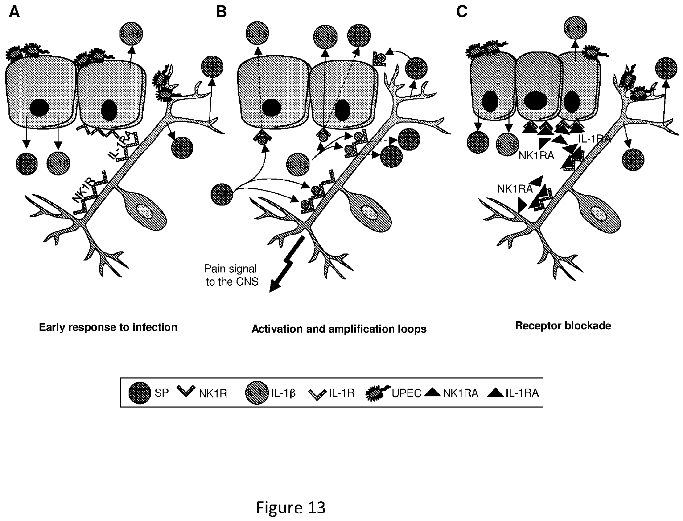

**Figure 13****. Model of the neuropeptide response to bladder infection**
   To summarize our *in vitro* and *in vivo* findings, we created a model showing the regulation of NK1R and SP in acute cystitis. **(A)** Model depicting the mechanism of action of bacterial activation of SP/NK1R and IL-1β. As bacteria bind to the bladder epithelial cells, SP and IL-1β expression increases and is secreted. In the nerve cells, bacterial infection causes an upregulation of NK1R, and SP expression is increased and secreted. **(B)** Following exogenous treatment, SP trigger an IL-1β response in bladder epithelial cells but not in nerve cells. IL-1β in turn activates SP/NK1R expression in both nerve- and epithelial cells. (C) By treating the cells with IL-1RA or SR140333 both the pain signal and the inflammatory signal are inhibited, as NK1R cannot be activated by SP, and IL-1R cannot be activated by IL-1β.

### Detailed Description of the Invention

### Example 1

### Neuropeptide response repertoire of infected nerve cells and bladder epithelial cells

Acute cystitis is initiated when bacteria engage in a crosstalk with epithelial cells and trigger a mucosal innate immune response, driven by IL-1β. The symptoms of acute cystitis suggest that nerve cells are actively involved, as well. To study the molecular basis of pain and involuntary muscle contractions, the applicants first performed a genome-wide screen for UTI-associated transcriptional responses in patients with bacteriuria.

Peripheral blood was harvested from five patients undergoing therapeutic inoculation with the genetically modified ABU strain *E. coli* 83972*fim.* Samples were obtained before and 3, 24 and 48 hours, and 1 and 2 weeks after inoculation. RNA was purified by QIAamp RNA blood Mini Kit (Qiagen), amplified with GeneChip 3' IVT Express Kit (Affymetrix) and hybridized onto Human Genome U219 arrays (Affymetrix) by AROS applied biotechnology (Århus, Denmark) or in-house using the Geneatlas system (Affymetrix). The data was normalized with Robust Multi Average (RMA) implemented in the Partek Express Software (Partek). Differentially expressed genes compared to pre-inoculation sample in each patient were analyzed using the Ingenuity Pathway Analysis (IPA) software (Qiagen). Regulated genes were then analyzed by Gene Set Enrichment Analysis (GSEA, ref.) using the c5.all.v.4.0 gene set collection in the GSEA desktop application (www.broadinstitute.org/gsea).

Genes encoding neuropeptide receptors and their ligands were upregulated, compared to infection-free intervals in the same patients (Fig 1). By GSEA analysis of the total data set (cut off FC >2.0), we identified significantly regulated gene sets **(****Fig. 1a****).** The most strongly regulated genes included Tachykinin 1 receptor (also known as Neurokinin-1 receptor, NK1R), its ligand Substance P (SP), Somatostatin receptor 2 (SSR2), Galanin receptor 2 (GalR2), Orexin (OR2C) and the taste receptor TAS2 **(****Fig. 1b****).** The results suggest that bacteria regulate the expression of neuropeptides and neuropeptide receptors. Galanin and its receptors, GALRs 1-3, all of which were regulated to some extent, are involved in nociception and pain signalling.

### Example 2

### Cellular Basis of Neuropeptide and Neuropeptide Receptor Response

To study the cellular basis of this response, nerve cells and epithelial cells were infected with acute cystitis isolates *in vitro* and characterized the effects on the neuropeptide receptor repertoire of these cells.

The cystitis strain CY-17 was isolated during a prospective study of childhood UTI in Gothenburg, Sweden. Bacteria were cultured on tryptic soy agar plates (TSA, 16 h, 37°C), harvested in phosphate buffered saline (PBS, pH 7.2) and diluted to appropriate concentration (CFU/ml) for infection.

We selected the neuronal cell line SH-SY5Y as well as the HTB9 human bladder epithelial cell line, which has been extensively used to study the molecular basis of acute cystitis.

Cells were grown on 8-well chamber slides (6×10⁴ cells/ well, Thermo Fischer Scientific) or in 6-well plates (6×10⁵ cells/well) for 16 hours in media supplemented with 10% FBS. Media were removed, and cells were washed with PBS before adding medium without FBS. Cells were infected with bacteria diluted to appropriate concentration in PBS and incubated for one or four hours at 37°C with 5% CO₂.

The two cell types were first infected with increasing concentrations of the acute cystitis strain *E. coli* CY-17 (10² - 10⁴ cfu/ml) for 1 to 4 hours. NK1R and SP expression was used as a read out of the cellular response. A time- and dose-dependent increase in NK1R and SP expression was observed in both cell types **(****Figure 1c****- e).** A concentration of 10⁴ cfu/ml of CY-17 and four hours of infection was chosen for further studies. Under these conditions, there was little cytotoxicity.

To further characterize the neuropeptide response to bladder infection, the *in vitro* analysis was extended to include GALR2, OrxR2 and TAS2R43. Confocal microscopy was used in this instance. Infected cells were fixed (3.7% formaldehyde, 15 min), permeabilized (0,25% triton X-100, 5% FBS, 10 min), blocked (5% FBS, 1h at RT), incubated overnight with primary antibodies in 5% FBS: Anti-NK1R (1:100, SC-15323, Santa Cruz), anti-SP, (1:200, ORB11399, Biorbyt), anti-orexin receptor 2, (1:200, AB10470, Abcam), anti-TAS2R43, (1:200, AB65513, Abcam), and anti-GALR2 antibodies (1:100, AB136264, Abcam). Appropriate fluorescently labeled secondary antibodies were used (Alexa Fluor^{®} 488 goat anti-rabbit IgG, A-11034; Life Technologies, (1hour at Room Temperature). After nuclear staining (DRAQ5, 1:1000, Abcam) slides were mounted (Fluoromount, Sigma-Aldrich), imaged by laser scanning confocal microscopy (MSM510 META confocal microscope, Carl Zeiss) and quantified by ImageJ software 1.46r (NIH).

This work showed a marked increase in GALR2 expression and a decrease in OrxR2 expression in infected SY5Y cells, while TAS2R43 remained unchanged compared to uninfected control cells (p< 0.001 and p < 0.001 respectively). The infected epithelial cells showed a similar pattern. These effects of CY-17 infection were confirmed by western blot analysis of whole cell extracts. NK1R, SP and GALR2 protein levels were elevated and OrxR2 levels were reduced in SH-SY5Y and HTB9 cells

The results suggest that acute cystitis strains activate neuropeptides- and neuropeptide receptors in both nerve cells and epithelial cells, with a similar response repertoire. GalR2 and OrxR2 have not previously been identified in the urinary tract.

### Example 3

### Influence of bacterial virulence on the neuropeptide response

An examination to see if the neuropeptide response was specific for acute cystitis strains or a general feature of urinary tract *E*. *coli* isolates was then carried out. Nerve cells and epithelial cells were exposed to CY-17, and controls in the form of the acute pyelonephritis (APN) strain CFT073 and the asymptomatic bacteriuria (ABU) strain *E. coli* 83972 as described in Example 2. The neuropeptide response was quantified by confocal microscopy also as described in Example 2.

The results were verified by western blot analysis of whole cell extracts. Infected cells were lysed with NP-40 lysis buffer supplemented with protease and phosphatase inhibitors (Roche Diagnostics). Whole cell lysates were run on SDS-PAGE (4-12% Bis-Tris gels, Invitrogen), blotted onto PVDF membranes (GE Healthcare), blocked with 5% BSA or 5% non-fat dry milk (NFDM) and incubated with primary antibodies: rabbit anti-NK1R (1:1000, SC-15323 Santa Cruz), mouse anti-Substance-P (1:1000, AB14184, Abcam, 1:1000), rabbit anti-orexin receptor 2 (1:1000, AB 10470, Abcam), rabbit anti-TAS2R43 (1: 1000 AB65513, Abcam) or rabbit anti-GALR2 (1:1000, AB136264, Abcam). The blots were washed with PBS tween 0.1 % (PBST) and incubated with secondary antibodies in 5% NFDM (goat anti rabbit-HRP or goat anti-mouse-HRP, Cell Signaling). The blots were washed with PBS tween 0.1 % (PBST) and developed with ECL Plus detection reagent (GE Health Care). HRP-linked anti-GAPDH (1: 1 000, sc-25778, Santa Cruz) was used as the loading control. Bands were quantified by ImageJ.

Results are shown in Figures 2 and 3. CY-17 triggered more pronounced NK1R and SP responses in nerve cells than the APN or ABU strains (p < 0.01 and p < 0.001 respectively), **(****Figure 2a****-f).** NK1R expression was also more strongly regulated by CFT073 than by ABU in those cells (p < 0.01). Similar results were obtained in bladder epithelial cells, where CY-17 and CFT073 triggered higher NK1R and SP responses than the ABU strain (p < 0.001, p < 0.001 and p < 0.05 compared to uninfected cells), **(****Figure 3a****-f).** Surprisingly, nuclear accumulation of SP was detected especially in cells infected with CY-17 **(****Figure 2g****).** In contrast, the GALR2 response was CY-17 specific in bladder epithelial cells, and OrxR2 staining was inversely regulated, as it was increased by ABU but decreased by the virulent strains. TAS2R43 was weakly inhibited by ABU and CY-17 in nerve cells and by all strains in bladder epithelial cells **(****Figures 2h** **and** **3h****).**

The results identify the acute cystitis strain CY-17 as a more potent regulator of neuropeptide responses than the APN or the ABU strain.

### Example 4

### Neuropeptide and neuropeptide receptor response to bladder infection in vivo

To investigate if infection activates mucosal neuropeptide- and neuropeptide receptor responses *in vivo,* recently defined murine acute cystitis model (Ambite IP et al. Molecular Basis of Acute Cystitis Reveals Susceptibility Genes and Immunotherapeutic TargetsPLOS pathogens 2016) was used. Mice were bred and housed in the specific pathogen-free MIG animal facilities (Lund, Sweden) with free access to food and water. Female C57BL/6 mice or *Il1b*^{*-*/}*⁻, Nlrp3*^{*-*/}*⁻, Asc^{-l-}* mice were used for experiments at 9-15 weeks of age. The *Il1b*^{*-*/*-*} mice have recently been shown to be functionally defective for IL-1a.

Acute cystitis was established in C57BL/6 WT mice, by intravesical inoculation with CY-17 (10⁸cfu/ml). Mice were sacrificed after seven days and the neuropeptide response to infection was characterized by immunohistochemistry of frozen bladder tissue sections, using NK1R-, SP-, GALR2-, OrxR2- and TAS2R43-specific antibodies.

Tissues were embedded in O.C.T. compound (VWR) and 7-µm-thick fresh cryosections were mounted on positively charged microscope slides (Superfrost/Plus; Thermo Fisher Scientific) Sections were permeabilized (0.25% Triton X-100, 5% FBS), blocked (5% FBS) and stained: rabbit anti-NK1R (1:50, SC-15323, SantaCruz), rabbit anti-substance p (1:100, ORB11399, Biorbyt), rabbit anti-orexin receptor 2 (1:300, AB104701, Abcam), rabbit anti-GALR2 (1:50, AB136264, Abcam) or rabbit anti-TAS2R43 (1:100, AB65513, Abcam). Antibodies were appropriately labeled using goat anti-rabbit secondary antibodies conjugated to Alexa 488 (1:200 in 0,025% Triton X-100, 5% FBS) for 1h in RT before nuclear counterstaining using Draq5 (1:1000, Abcam). Sections were imaged by laser scanning confocal microscopy (MSM510 META confocal microscope, Carl Zeiss) or by fluorescence microscopy (AX60, Olympus Optical).

Results were confirmed by q-PCR analysis of whole bladder RNA from infected mice, Complementary DNA was reverse-transcribed from 0.2-1 µg of mice total RNA using SuperScript^{™} III First-Strand Synthesis System for RT-PCR (Invitrogen), and quantified in real time using primer pairs against *Mus Musculus Nk1r, Ppt-1 ³⁷*and *Galr2* and QuantiTect^{®} SYBR^{®} Green PCR kits (Qiagen) on a Rotor Gene Q (Qiagen). The primers used are shown in Table 1.

**Table 1**

| **Gene** | **Forward /reverse** | **Sequence** | **Product size (BP)** | **SEQ ID No** |
|---|---|---|---|---|
| *Nk1r* | Forward | 5'--CTTGCCTTTTGGAACCGTGTG--3' | 501 | 1 |
| | Reverse | 5'--CACTGTCCTCATTCTCTTGTGGG--3' | | 2 |
| *Ppt--1* | Forward | 5'--GCCAATGCAGAACTACGAAA--3' | 282 | 3 |
| | Reverse | 5'--GCTTGGACAGCTCCTTCATC--3' | | 4 |
| *Galr2* | Forward | 5'--TCACTGCTCTGCAAGGCCGTTCA--3' | 233 | 5 |
| | Reverse | 5'- AGATTGGCCAGCTGCGACTGACTGT-3' | | 6 |

qRT-PCR reactions were run in technical duplicates and gene expression was analyzed based on ΔΔCT comparison to *Mus Musculus* GAPDH.

The results are **(****Figure 4****).** Mucosal NK1R and SP expression increased after CY-17 infection (p < 0.05, **Figure 4a****)** but the effect on SP expression was less pronounced **(****Figure 4b****,** p=0.09). Interestingly, GALR2 staining increased dramatically **(****Figure 4C****,** p<0.001) and qRT-PCR analysis of whole bladder RNA confirmed these changes **(****Figure 4d****).** TAS2R43 and Orx2R expression was detected by immunohistochemistry but did not change in response to infection **(****Figure 4e**, **4f**).

The results demonstrate that infection alters the neuropeptide activation state in the bladder mucosa, with a pattern that is directly relevant to the symptoms that accompany acute cystitis.

### Example 5

### Cellular localization and production of neuropeptides and neuropeptide receptors

To further define the cellular origin of NK1R, SP and GALR2 **(****Figure 4****),** bladder tissue sections were stained with antibodies specific for nerve cells (βIII tubulin), epithelial cells (MN116), neutrophils (RB68C5) or macrophages (RM0029-11H3), with co-staining for NK1R, SP or GALR2.

In *Nlrp3*^{*-*/*-*} mice with severely inflamed bladders, βIII tubulin staining was increased compared to uninfected controls, with a pattern suggestive of nerve protrusions through the epithelial barrier, towards the lumen **(****Figure 5a****).** Co-localization of βIII tubulin with NK1R was clearly visible in *Nlrp3*^{*-*/*-*} mice, from the nerve cell plexus through the epithelial basal cell layer towards the mucosal lumen **(****Figure 5b****).** SP showed significant colocalization with βIII tubulin along the epithelial-nerve cell interface, but the staining was more restricted than for NK1R **(****Figure 5c****).** In contrast, the GalR2 response was mainly epithelial except for a few, stained cells inside the nerve plexus, which did not co-localize with beta tubulin **(****Figure 5c****).** In infected C57BL/6WT mice, the sub-epithelial nerve plexus was clearly delineated, with a more distinct border to the epithelium.

In *Nlrp3*^{*-*/*-*} mice, a strong, mucosal neutrophil response to infection was detected but the neutrophils did not stain for NK1R, SP or GALR2. Scattered macrophages were present in the sub-epithelial space of infected C57BL/6WT and *Nlrp3*^{*-*/*-*} mice, but showed no co-localization with NK1R, SP or GALR2 (not shown).

The results suggest that the infected epithelium and sub-epithelial nerve cell plexus both respond to mucosal infection and participate in direct, neuropeptide based interactions. Through NK1R and SP, the mucosal nerve cell plexus can also signal to the CNS, where pain is controlled.

### Example 6

### NK1R inhibition attenuates SP production and mucosal inflammation

The applicants subsequently inhibited the neuropeptide response, *in vivo,* using the NK1R antagonist SR140333 (Oury-Donat F, et al., Journal of neurochemistry 1994; 62(4): 1399-1407). This irreversible antagonist prevents SP from binding to its receptor.

Susceptible *Nlrp3*^{*-*/*-*} mice were treated with SR140333 intra-peritoneally (1 mg/kg), one hour before infection (pretreatment) or 30 minutes after infection (posttreatment) with CY-17 **(****Figure 6a****).** The severity of acute cystitis after 24 hours or seven days was examined and neuropeptide expression and disease severity quantified by immunohistochemistry and qRT-PCR of whole bladder RNA extracts, using primers specific for *NK1R, Ppt-1* or *Galr2* as described above. Results are shown in Figure 6(b)-(h)

SR140333 treatment reduced the mucosal NK1R and SP response to infection in epithelial cells and the mucosal nerve cell plexus **(****Figure 6c and 6e****).** *Nk1r* expression was reduced by prophylactic and post-infection treatment (p < 0.05 and p < 0.01, respectively) (Figure 7d). *Ppt-1* expression was affected by post-infection treatment (p < 0.01) but prophylaxis was less efficient (p = 0.06) **(****Figure 6f****).** There was no significant effect on *Galr2* expression by either treatment (p = 0.19 and p = 0.63 respectively) **(****Figure 6g, h**).

In addition, the treated *Nlrp3*^{*-*/*-*} mice showed a reduction in gross bladder pathology, defined by edema, hyperemia and bladder enlargement, compared to infected mice without therapy (24 hours post infection, **Figure 6b****).** Bladder tissue was more intact and the inflammatory cell infiltrate was reduced in tissues.

The results suggest that blocking of NK1R signaling reduces the NK1R and SP responses to infection. In addition, the inhibitor reduced the inflammatory response in infected bladders, suggesting that NK1R controls innate immunity. The findings predict that SR140333 treatment would reduce pain signaling mediated by SP and NK1R but not by GALR2.

The effects of NK1R inhibition were confirmed *in vitro* using CY-17 infected human bladder cells. To validate the *in vivo* inhibition results described above and to understand the relative contribution of nerve and bladder epithelial cells in the model, the NK1R inhibitor (SR140333) and the IL-1receptor antagonist (IL-1RA) were used *in vitro.*

HTB-9 cells and differentiated SH-SY5Y cells were pre-treated with SR140333, or IL-1RA Anakinra (500 ng/ml) for 30 minutes before infection with CY17 (10⁴ cfu/ml, four hours), or treatment with exogenous recombinant SP (R&D System) or IL-1β (Abnova) (20 ng/ml, four hours). After infection, cells were infected with CY17 and the NK1R, SP and IL-1β response was quantified by confocal imaging. IL-1β- and SP secretion was quantified by ELISA, in culture supernatants. The results are shown in **Figure 10****.** In nerve- and bladder epithelial cells, SR140333 inhibited the SP/NK1R response to infection, consistent with an autocrine activation loop involving SP/NK1R as shown by confocal imaging **(****Figure 10A****).** The nerve cells did not produce IL-1β and IL-1RA had no effect on the SP/NK1R response during infection **(****Figure 10B****).** These results were confirmed by ELISA showing a decrease in SP secretion in both nerve- and epithelial cells. IL-1β secretion was inhibited in epithelial cells by both SR140333 and IL-1RA **(**Figure 10 C-D) Exogenous IL-1β increased SP/NK1R in both cell types, suggesting that IL-1 receptors are expressed by activated nerve cells and that IL-1β activates SP/NK1R expression. SP activated IL-1β expression and secretion in bladder epithelial cells but had no effect on IL-1β on nerve cells. (^{∗} = *P* < 0.05, ** = *P* < 0.01, *** = *P* < 0.001, Two-tailed t-test for confocal imaging data and Mann-Whitney U-test for ELISAs.

Furthermore, to examine if the effects of NK1R inhibition can be generalized, we used two additional NK1R antagonists (CP99994 and L703.303). HTB-9 cells and differentiated SH-SY5Y cells were pre-treated with SR140333, CP99994, L-703.606 or IL-1RA Anakinra (500 ng/ml) for 30 minutes before infection with CY17 (10⁴ cfu/ml, four hours), or treatment with exogenous recombinant SP (R&D System) or IL-1β (Abnova) (20 ng/ml, four hours).

Pretreatment of bladder epithelial cells with these inhibitors prior to CY17 infection resulted in a similar reduction in NK1R and SP expression as with SR140333 (Figure 11A-B). The two NK1R antagonists also reduced the epithelial IL-1β response to infection (Figure 11E). The results link NK1R activation to the inflammatory response in infected bladders, and to acute cystitis.

### Example 7

### Genetic control of the neuropeptide- and neuropeptide receptor responses

This strong effect on mucosal inflammation suggested that NK1R and SP also regulate innate immune responses involved in bladder inflammation. It has recently been reported that acute cystitis, as a hyper-inflammatory state of the bladder mucosa, is driven by an imbalanced IL-1β response. Genetic defects affecting the inflammasome constituents *Asc* and *Nlrp3* were shown to create a dysregulated inflammatory state, with overexpression of the metalloproteinase MMP7 and excessive, inflammasome independent pro-IL-1β processing. As a consequence, *Il1b*^{*-*/*-*} mice were protected from acute cystitis and C57BL/6 WT mice developed a moderate, self-limiting disease.

To examine if the neuropeptide- and neuropeptide-receptor responses are also controlled by this mechanism, *Asc*^{*-*/*-*} or *Nlrp3*^{*-*/*-*} mice were infected with CY-17 as described above, and the severity of acute cystitis after seven days was determined. The response was quantified by immunohistochemistry and confirmed by qRT-PCR of RNA (as described above) extracted from whole bladder tissue, compared to C57BL/6 WT mice and uninfected controls. The results are shown in Figure 7.

Major, genotype-specific differences in neuropeptide staining were detected. The susceptible *Nlrp3*^{*-*/*-*} and *Asc^{-l-}* mice with bladder pathology showed a marked increase in mucosal NK1R staining, with a predominance of epithelial- and some sub-epithelial staining (p < 0.01 and 0.001 compared to infected C57BL/6 WT mice). The mucosal SP response showed a similar pattern (p < 0.01 and p < 0.05 respectively), **(****Figure 7a****-d).** GALR2 expression was also activated in *Nlrp3*^{*-*/*-*} and *Asc*^{*-*/*-*} mice (p<0.001 compared to C57BL/6 WT mice) and epithelial staining was accompanied by a subepithelial, punctate staining pattern, possibly representing nerve cell somas. Urine SP levels were elevated in infected C57BL/6 WT mice compared to uninfected controls (217 pg/ml compared to 43 pg/ml, *P* < 0.01) and in infected *Asc*^{*-*/*-*} and *Nlrp3*^{*-*/*-*} mice (129 pg/ml and 142 pg/ml respectively, *P* < 0.05).

The tissue sections were also stained for neutrophils or macrophages. The infiltrating neutrophils in *Nlrp3*^{*-*/*-*} mice did not stain for NK1R or SP. Scattered sub-epithelial macrophages in infected C57BL/6WT- and *Nlrp3*^{*-*/*-*} mice showed no staining for NK1R or SP. The genetic control of the neuropeptide response was further analyzed, by infection of *l1b*^{*-*/*-*} and *Tlr4*^{*-*/*-*} mice. Consistent with the importance of IL-1β for acute cystitis, *Il1b*^{-/-} mice were protected from pathology and showed a weak mucosal NK1R response to infection. SP levels were higher in *Il1b*^{*-*/*-*} mice than in C57BL/6WT mice, suggesting an IL-1β independent response mechanism, but was much lower than in *Asc*^{*-*/*-*}or *Nlrp3*^{*-*/*-*} mice (p<0.05, **Figure 7c**, **d**). Importantly, this response was *Tlr4* dependent, as *Tlr4*^{*-*/*-*} mice showed no NK1R or SP response (data not shown). In contrast, GALR2 expression was activated to the same extent in *Tlr4*^{*-*/*-*} and *Il1b*^{*-*/*-*} mice as in C57BL/6WT mice.

The results suggest that the NK1R and SP response to *E. coli* infection is controlled by *Tlr4* in both nerve cells and bladder epithelial cells. In addition, the mucosal hyper-activation state in *Nlrp3*^{*-*/*-*} and *Asc*^{*-*/*-*} mice involved *Il1b.* The results illustrate the cross-talk between IL-1β- and neuropeptide signaling, leading to mutual control of mucosal inflammation. Importantly, the GalR2 response was not controlled by this mechanism. Thus it can be concluded that TLR4 and IL-1β are upstream regulators of mucosal NK1R and SP responses and *Asc* and *Nlrp3* are determinants of exaggerated NK1R and SP expression in acute cystitis.

### Example 8

### The IL-1receptor (IL-1R) antagonist Anakinra prevents acute cystitis and inhibits the SP and NK1R response

To confirm the importance of *Il1b* for the neuropeptide response, susceptible *Asc^{-l-}*mice were treated with the IL-1 receptor (IL-1R) antagonist Anakinra^{RTM} **(****Figure 8a****).** This therapeutic approach was recently shown to be effective against acute cystitis, as Anakinra^{RTM} attenuated the hyper-inflammatory response to infection and prevented bladder pathology in susceptible mice.

The results were obtained as described above and are shown in **Figure 8****.** A marked reduction in NK1R and SP responses was observed in Anakinra^{R} treated mice compared to infected controls without therapy. Furthermore, the baseline NK1R and SP staining was reduced by Anakinra^{R} in uninfected mice, suggesting that *Il1b* regulates basic neuropeptide levels in the bladder mucosa. These findings were confirmed *in vitro* by pretreating nerve cells and bladder epithelial cells with Anakinra (500 ng/ml) followed by CY-17 infection (10⁴ CFU/ml, 4 hours). We detected a marked reduction in NK1R staining after Anakinra treatment, in both nerve cells and bladder epithelial cells.

The findings link the mucosal neuropeptide and neuropeptide receptor responses to IL-1β and to a new mechanism of IL-1b activation and processing by MMP-7, which is responsible for in the pathogenesis of acute cystitis in *Asc*^{*-*/*-*} and *Nlrp3*^{*-*/*-*} mice.

### Example 9

### Elevated substance P levels in urine from patients with acute cystitis

Urine samples were obtained for SP analysis. Samples from patients with community acquired acute cystitis were collected at two primary health care units in Lund, Sweden. Urine samples were also collected from patients suffering with ABU. Urine SP levels were quantified by ELISA using Human Substance P ELISA kit (ab133029) (Abcam).

Diagnosis of acute cystitis was based on a urine dipstick analysis positive for bacteria and lower urinary tract symptoms (dysuria and suprapubic pain and without fever). Urine samples were obtained from a subset of nine patients, in whom asymptomatic carriage and symptomatic episodes were recorded.

In addition, patients with ABU who had participated in a therapeutic inoculation trial with *E. coli* 83972 were enrolled and enrolment was based on incomplete bladder emptying and recurrent symptomatic UTIs. Therapeutic inoculation is a therapeutic alternative to preventing symptomatic infection and the safety and efficacy of this approach has been validated in placebo-controlled clinical studies. Samples were obtained from inoculated patients, who carried the ABU strain *E*. *coli* 83972 asymptomatically, for long periods of time (months-years).

Symptomatic episodes were recorded in the control arm of the study or after spontaneous clearance of *E. coli* 83972. 40 ABU urine samples were selected for analysis from 20 patients and 28 urine samples during symptomatic episodes from 12 patients. Samples were selected based on high scoring during symptomatic episodes, and from normal ABU samples.

To address if SP expression is elevated in patients with acute cystitis, the SP concentrations in urine samples obtained at the time of diagnosis (positive dipstick and lower urinary tract symptoms without fever, *n*=9) were quantified. Urine samples were also collected from patients who developed long-term ABU following therapeutic inoculation with the ABU strain *E. coli* 83972 *(n* = 29). The acute cystitis patients had significantly higher urine SP levels than patients with ABU (126 pg/ml vs 75.5, p < 0.01) **(****Figure 9a, b**).

While therapeutic inoculation with *E*. *coli* 83972 is protective, a few patients developed symptoms from the urinary tract during long-term follow up *(n* = 12). Therefore paired urine samples were collected during asymptomatic carriage and at the time of symptomatic flares in patients with ABU and compared the urine SP levels in individual patients. All but one patient had higher urine SP levels during symptoms than during ABU (191 pg/ml vs 109 pg/ml, p < 0.01, **Figure 9c****).**

During this study, an association between the neurological status of the patients and the urine SP levels was noted. Therapeutically inoculated patients included one group with spinal lesions and one with intact bladder innervation, who were prone to recurrent UTIs due to residual urine.

The increase in urine SP levels during symptomatic episodes was not detected in patients with spinal lesions (185 pg/m vs 56 pg/ml, p < 0.05) as no increase (113 pg/ml vs 98 pg/ml, p>0.05) **(****Figure 9d****).**

The results show that acute cystitis triggers a SP response, which can be detected in urine. As the distal nerves innervating the bladder undergo degeneration after spinal lesions, the results also suggest that the SP response requires intact bladder innervation.

### Example 10

### Transcriptional regulation of NK1R and SP expression

*ASC* and *NLRP3* were recently identified as transcriptional repressors of MMP7; a protease responsible for non-canonical processing of pro-IL-1β in hosts lacking a functional inflammasome. The over-activation of SP/NK1R in *Nrp3*^{*-*/*-*} mice suggested that a similar mechanism might regulate SP/NK1R expression. To address this question, we transfected bladder epithelial cells *in vitro* with *ASC-* or *NLRP3*-specific siRNAs (24 hours). Specifically, HTB-9 cells were transfected with PYCARD/ASC- and NLRP3-specific siRNAs (0.09 µM, Flexi-Tube GeneSolution, #GS29108 and #GS114548, Qiagen) or with AllStars Negative Control siRNA (#SI03650318, Qiagen) using the HiPerFect Transfection Reagent (#301705, Qiagen) for 17 hours.

The transfected cells were then infected with CY17 (10⁴ CFU/ml, 4 hours) and analyzed for changes in SP/NK1R expression by immunostaining. The results are shown in **Figure 12****.** NK1R and SP staining increased by about 70% in cells transfected with *ASC-* or *NLRP3*-specific siRNA **(****Figure 12A****).** The effect was further enhanced by infection, compared to the scrambled siRNA control or non-transfected cells (*P* < 0.01 for *NLRP3-* or *ASC*-siRNA transfected cells, respectively), (**Figure 12A-D**). Transfection decreased *ASC*- and NLRP3-expression and a further reduction occurred after infection, as detected by confocal imaging and western blots **(****Figure 12E****).**

The results suggest that *Nlrp3* and *Asc* may act as repressors of NK1R expression in uro-epithelial cells (see model in **Figure 12F****).** To address this question, we examined if *ASC-* and *NLRP-3* bind to the *TACR1* promoter **(****Figure 12G****).** Lysates from uninfected HTB9 cells were added to a *TACR1* promoter fragment of 214 base pairs, creating a triple band shift, in an Electro Mobility Shift Assay (a, b and c, **Figure 12H****).** Antibodies to NLRP-3 removed band a and attenuated band b and antibodies to ASC reduced band a. Both bands a and b were attenuated by anti-NLRP-3- and anti-ASC antibodies, in combination, consistent with the ASC-dependence of NLRP-3. The band shifts resulted in a loss of free probe.

The model in **Figure 13** illustrates how infection activates SP and IL-1β expression in bladder epithelial cells and SP and NK1R expression in nerve cells **(****Figure 13A****).** The secreted molecules and their receptors then participate in an amplification loop, further increasing nociception though NK1R/SP and inflammation through IL-1β /IL-1R activity **(****Figure 13B****).** These effects were blocked in the presence of NK1R inhibitors or the IL-1R antagonist, respectively **(****Figure 13C****).**

## Claims

1. An antagonist of Neurokinin-1 receptor (NK1R), or its ligand Substance P (SP), for use in the treatment of bacterial infection or for the management of pain caused by bacterial infections.

2. An antagonist for use according to claim 1 wherein the bacterial infection is cystitis.

3. An antagonist for use according to any one of the preceding claims which is an antagonist of NK1R.

4. An antagonist for use according to claim 3 which is selected from SR140333 (1-[2-[(3*S*)-3-(3,4-Dichlorophenyl)-1-[2-[3-(1-methylethoxy)phenyl]acetyl]-3-piperidinyl]ethyl]-4-phenyl-1-azoniabicyclo[2.2.2]octane chloride), Aprepitant (5-([(2*R*,3*S*)-2-((*R*)-1-[3,5-bis(trifluoromethyl)phenyl]ethoxy)-3-(4-fluorophenyl)morpholino]methyl)-1*H*-1,2,4-triazol-3(2*H*)-one), Fosaprepitant ([3-{[(2*R*,3*S*)-2-[(1*R*)-1-[3,5-bis(trifluoromethyl)phenyl] ethoxy]-3-(4-fluorophenyl)morpholin-4-yl]methyl}-5-oxo-2*H*-1,2,4-triazol-1-yl]phosphonic acid), Casopitant ((2*S*,4*S*)-4-(4-Acetyl-1-piperazinyl)-*N*-[(1*R*)-1-[3,5-bis(trifluoromethyl)phenyl]ethyl]-2-(4-fluoro-2-methylphenyl)-N-methyl-1-piperidinecarboxamide), L-733,060 ((2*S*,3*S*)-3-{[3,5-bis(trifluoromethyl)benzyl]oxy}-2-phenylpiperidine), Vestipitant (2*S*)-*N*-[(1*R*)-1-[3,5-bis(trifluoromethyl)phenyl]ethyl]-2-(4-fluoro-2-methylphenyl)-*N-*methylpiperazine-1-carboxamide), Maropitant ((7*R*,8*S*)-*N*-[(5-*tert*-Butyl-2-methoxyphenyl)methyl]-7-[di(phenyl)methyl]-1-azabicyclo[2.2.2]octan-8-amine CP99994 ((2*S*,3*S*)-*N*-[(2-Methoxyphenyl)methyl]-2-phenyl-3-piperidinamine) and L703.606 ((2R,3R)-2-benzhydryl-N-[(2-iodanylphenyl)methyl]-1-azabicyclo[2.2.2]octan-3-amine).

5. An antagonist for use according to claim 4 which is SR140333 CP99994 ((2*S*,3*S*)-*N*-[(2-Methoxyphenyl)methyl]-2-phenyl-3-piperidinamine) or L703.606 ((2R,3R)-2-benzhydryl-N-[(2-iodanylphenyl)methyl]-1-azabicyclo[2.2.2]octan-3-amine).

6. An antagonist for use according to claim 5 which is SR140333.

7. A combination comprising an antagonist for the use of any one of claims 1 to 6 and a further therapeutic agent.

8. The combination for the use of claim 7 wherein the further therapeutic agent is an IL-1β inhibitor, an MMP inhibitor, or a protein selected from ASC or NLRP-3.

9. The combination for the use of claim 8 wherein the further therapeutic agent is anakinra.

10. A pharmaceutical composition comprising an antagonist for use according to any one of claims 1 to 6 or a combination according to any one of claims 7 to 9.

11. A method for diagnosing cystitis and in particular, acute cystitis, said method comprising detecting elevated levels of SP in urine of a subject.

## Patentansprüche

1. Antagonist des Neurokinin-1-Rezeptors (NK1R) oder seines Liganden Substanz P (SP) zur Verwendung bei der Behandlung einer bakteriellen Infektion oder zur Behandlungsplanung durch bakterielle Infektionen verursachter Schmerzen.

2. Antagonist zur Verwendung nach Anspruch 1, wobei die bakterielle Infektion Zystitis ist.

3. Antagonist zur Verwendung nach einem der vorhergehenden Ansprüche, der ein Antagonist von NK1R ist.

4. Antagonist zur Verwendung nach Anspruch 3, der ausgewählt ist aus SR140333 (1-[2-[(3S)-3-(3,4-Dichlorphenyl)-1-[2-[3-(1-methylethoxy)phenyl]acetyl]-3-piperidinyl]ethyl]-4-phenyl-1-azoniabicyclo[2.2.2]octanchlorid), Aprepitant (5-([(2R,3S)-2-((R)-1-[3,5-Bis(trifluormethyl)phenyl]ethoxy)-3-(4-fluorphenyl)morpholino]methyl)-1H-1,2,4-triazol-3(2H)-on), Fosaprepitant ([3-{[(2R,3S)-2-[(1R)-1-[3,5-Bis(trifluormethyl)phenyl]ethoxy]-3-(4-fluorphenyl)morpholin-4-yl]methyl}-5-oxo-2H-1,2,4-triazol-1-yl]phosphonsäure), Casopitant ((2S,4S)-4-(4-Acetyl-1-piperazinyl)-N-[(1R)-1-[3,5-bis(trifluormethyl)phenyl]ethyl]-2-(4-fluor-2-methylphenyl)-N-methyl-1-piperidincarboxamid), L-733,060 ((2S,3S)-3-{[3,5-Bis(trifluormethyl)benzyl)]oxy}-2-phenylpiperidin), Vestipitant (2,S)-N-[(1R)-1-[3,5-Bis(trifluormethyl)phenyl]ethyl]-2-(4-fluor-2-methylphenyl)-N-Methylpiperazin-1-carboxamid), Maropitant ((7R,8S)-N-[(5-tert-Butyl-2-methoxyphenyl)methyl]-7-[di(phenyl)methyl]-1-azabicyclo[2.2.2]octan-8-amin, CP99994 ((2S,3S)-N-[(2-Methoxyphenyl)methyl]-2-phenyl-3-piperidinamin) und L703.606 ((2R,3R)-2-Benzhydryl-N-[(2-iodanylphenyl)methyl]-1-azabicyclo[2.2.2]octan-3-amin).

5. Antagonist zur Verwendung nach Anspruch 4, der SR140333, CP99994 ((2S,3S)-N-[(2-Methoxyphenyl)methyl]-2-phenyl-3-piperidinamin) oder L703.606 ((2R,3R)-2-Benzhydryl-N-[(2-iodanylphenyl)methyl]-1-azabicyclo[2.2.2]octan-3-amin) ist.

6. Antagonist zur Verwendung nach Anspruch 5, der SR140333 ist.

7. Kombination, umfassend einen Antagonisten zur Verwendung nach einem der Ansprüche 1 bis 6 und ein weiteres therapeutisches Mittel.

8. Kombination zur Verwendung nach Anspruch 7, wobei das weitere therapeutische Mittel ein IL-1β-Inhibitor, ein MMP-Inhibitor oder ein aus ASC oder NLRP-3 ausgewähltes Protein ist.

9. Kombination zur Verwendung nach Anspruch 8, wobei das weitere therapeutische Mittel Anakinra ist.

10. Pharmazeutische Zusammensetzung, umfassend einen Antagonisten zur Verwendung nach einem der Ansprüche 1 bis 6 oder eine Kombination zur Verwendung nach einem der Ansprüche 7 bis 9.

11. Verfahren zur Diagnose von Zystitis und insbesondere akuter Zystitis, wobei das Verfahren den Nachweis erhöhter SP-Spiegel im Urin eines Patienten umfasst.

## Revendications

1. Antagoniste du récepteur de la neurokinine-1 (NK1R), ou de son ligand substance P (SP), destiné à être utilisé dans le traitement d'une infection bactérienne ou pour la gestion de la douleur causée par des infections bactériennes.

2. Antagoniste destiné à être utilisé selon la revendication 1, ladite infection bactérienne étant la cystite.

3. Antagoniste destiné à être utilisé selon l'une quelconque des revendications précédentes, qui est un antagoniste de NK1R.

4. Antagoniste destiné à être utilisé selon la revendication 3, qui est choisi parmi le SR140333 (chlorure de 1-[2-[(3*S*)-3-(3,4-dichlorophényl)-1-[2-[3-(1-méthyléthoxy)phényl]acétyle]-3-pipéridinyl]éthyl]-4-phényl-1-azoniabicyclo[2.2.2]octane), l'aprépitant (5-([(2*R*,3*S*)-2-((*R*)-1-[3,5 -bis(trifluorométhyl)phényl]éthoxy)-3-(4-fluorophényl)morpholino]méthyl)-1*H-*1,2,4-triazol-3(2*H*)-one), le fosaprépitant (l'acide [3-{[(2*R*, 3*S*)-2-[(1*R*)-1-[3,5-bis(trifluorométhyl)phényl] éthoxy]-3-(4-fluorophényl)morpholin-4-yl]méthyl}-5-oxo-2*H*-1,2,4-triazol-1-yl]phosphonique), le casopitant ((2*S*,4*S*)-4-(4-acétyl-1-pipérazinyl)-*N*-[(1*R*)-1-[3,5-bis(trifluorométhyl)phényl]éthyl]-2-(4-fluoro-2-méthylphényl)-N-méthyl-1-pipéridinecarboxamide), le L-733,060 ((2*S*,3*S*)-3-{[3,5-bis(trifluorométhyl)benzyl]oxy}-2-phénylpipéridine), le vestipitant (2*S*)-*N*-[(1*R*)-1-[3,5-bis(trifluorométhyl)phényl]éthyl]-2-(4-fluoro-2-méthylphényl)-*N-*méthylpipérazine-1-carboxamide), le maropitant ((7*R*,8*S*)-*N*-[(5-*tert*-butyl-2-méthoxyphényl)méthyl]-7-[di(phényl)méthyl]-1-azabicyclo[2.2.2] octan-8-amine, le CP99994 ((2*S*,3*S*)-*N*-[(2-méthoxyphényl)méthyl]-2-phényl-3-pipéridinamine) et le L703.606 ((2R,3R)-2-benzhydryl-N-[(2-iodanylphényl)méthyl]-1-azabicyclo[2.2.2]octan-3-amine).

5. Antagoniste destiné à être utilisé selon la revendication 4, qui est le SR140333, le CP99994 ((2*S*,3*S*)-*N*-[(2-méthoxyphényl)méthyl]-2-phényl-3-pipéridinamine) ou le L703.606 ((2R,3R)-2-benzhydryl-N-[(2-iodanylphényl)méthyl]-1-azabicyclo[2.2.2]octan-3-amine).

6. Antagoniste destiné à être utilisé selon la revendication 5, qui est le SR140333.

7. Combinaison comprenant un antagoniste destiné à être utilisé selon l'une quelconque des revendications 1 à 6 et un agent thérapeutique supplémentaire.

8. Combinaison destinée à être utilisée selon la revendication 7, ledit agent thérapeutique supplémentaire étant un inhibiteur de IL-1β, un inhibiteur de MMP ou une protéine choisie parmi ASC ou NLRP-3.

9. Combinaison destinée à être utilisée selon la revendication 8, ledit agent thérapeutique supplémentaire étant l'anakinra.

10. Composition pharmaceutique comprenant un antagoniste destiné à être utilisé selon l'une quelconque des revendications 1 à 6 ou une combinaison selon l'une quelconque des revendications 7 à 9.

11. Procédé permettant de diagnostiquer une cystite et en particulier une cystite aiguë, ledit procédé comprenant la détection de taux élevés de SP dans l'urine d'un sujet.
